Europäisches Patentamt

(19) European Patent Office

Office européen des brevets

(11) Publication number: **0 232 994**
**B1**

(12) **EUROPEAN PATENT SPECIFICATION**

(45) Date of publication of the patent specification:
**19.12.90**

(21) Application number: **87300533.4**

(22) Date of filing: **22.01.87**

(51) Int. Cl.⁵: **A61M 25/01**

(54) **Catheter Introducer.**

(30) Priority: **04.02.86 JP 22704/86**

(43) Date of publication of application:
**19.08.87 Bulletin 87/34**

(45) Publication of the grant of the patent:
**19.12.90 Bulletin 90/51**

(84) Designated Contracting States:
**AT BE CH DE ES FR GB IT LI LU NL SE**

(56) References cited:
**EP-A- 0 141 006**
**DE-B- 2 005 167**
**GB-A- 2 033 236**

(73) Proprietor: **SHERWOOD MEDICAL COMPANY,**
**1831 Olive Street, St. Louis, MO 63103(US)**

(72) Inventor: **Okada, Yosuke, 2748-1, Ichinomiya**
**Mori-Machi, Shuchi Gun Shizuoka Pref.(JP)**

(74) Representative: **Porter, Graham Ronald et al, c/o Wyeth**
**Laboratories Huntercombe Lane South, Taplow**
**Maidenhead Berkshire SL6 0PH(GB)**

ACTORUM AG

## Description

This invention relates to a catheter introducer.

A catheter introducer generally comprises a tubular member having a conical tip and is used in the procedure for insertion of a medical catheter (see for example GB-A 2 033 236). In use, the conical tip of the introducer is inserted into a puncture wound to dilate the wound opening so that a sheath of the introducer can be pushed into the wound; when the sheath is inserted the introducer is removed for disposal. The sheath provides a passageway for a medical catheter and is also removed for disposal once the catheter is inserted. The introducer may have an internal guide wire, for example where the introducer is intended for insertion into a blood vessel. It is also known to provide the guide wire with a flexible distal portion of reduced diameter, cf. EP-A 0 141 006.

One problem with wire guided introducers is that the guide wire may buckle because the tip of the introducer is very rigid when compared with the wire; the wire is necessarily flexible to avoid damage to body tissue. If the wire buckles, as will be further described hereinafter, it is impossible to insert the introducer correctly and moreover, the buckled guide wire may damage tissue especially as it is withdrawn. The user may be unable to distinguish resistance due to a buckled guide wire from the general resistance offered by the body tissue.

It is an object of the invention to provide a catheter introducer which overcomes the aforementioned disadvantages.

According to the invention there is provided a catheter introducer for use with a guide wire and comprising a tubular body having an inwardly tapered tip, said tip having a flexible snout adapted to follow the path of a guide wire.

The flexible snout ensures that the introducer is guided into a puncture wound before the user applies additional force to dilate the opening. A further feature of the invention is that a smaller diameter guide wire may be used because buckling is less likely; this has the advantage that if the guide wire is misplaced or inadvertently punctures a blood vessel wall less damage and trauma is caused to the patient.

Preferably the snout is integrally formed with the tip of the introducer and is of the same material. In one embodiment of the invention the snout has a substantially constant outside diameter. In an alternative embodiment the snout has a slight outward taper in a proximal direction; the taper being substantially less than the conical tip of the introducer which is used to dilate the wound opening. The snout may have a substantially constant wall thickness.

The invention also provides a catheter introducer assembly comprising the introducer aforesaid, a guide wire and a sheath.

Other features of the invention will be apparent from the following description of two preferred embodiments shown by way of example only in the accompanying drawings in which:-

Figure 1 is a plan view of a prior art introducer with guide wire and sheath;

Figure 2 is an axial section through the tip of a prior art introducer;

Figure 3 illustrates buckling of a guide wire during insertion of a prior art introducer;

Figure 4 is an axial section through the tip of one introducer according to the present invention;

Figure 5 is an axial section through the tip of a second introducer according to the present invention;

Figure 6 illustrates insertion of the introducer of Figure 4 over a guide wire; and

Figure 7 is a graph illustrating relative stiffness of the tip of the introducers of Figures 2, 4 and 5.

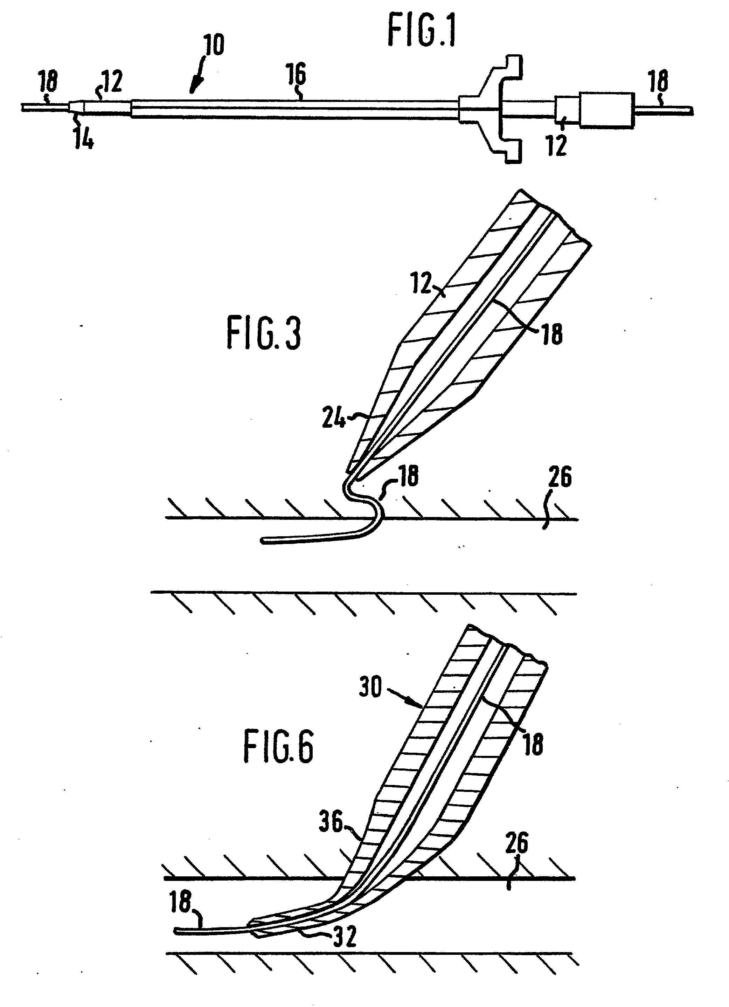

With reference to Figure 1 of the drawings there is illustrated a prior art catheter introducer 10 comprising a tubular body 12 having a generally conical distal end or dilator 14. The introducer has a sheath 16 and a guide wire 18.

Figure 2 shows the dilator in greater detail. The bore 20 of the introducer reduces in diameter to provide a distal opening through which the guide wire is a sliding fit. The tip of the dilator is a circular sharp edge 22 to provide a smooth transition from the guide wire to the conical surface 24. The wall thickness of the dilator decreases slightly at the tip thereof by virtue of the edge 22.

Figure 3 illustrates inadvertent buckling of a guide wire during insertion of the introducer of Figure 2. After insertion of the guide wire 18, into for example, a blood vessel 26, the introducer is pushed through the puncture wound. If the user does not choose the correct attack angle for the introducer 12, the guide wire may be buckled as shown. Further inward movement of the introducer will only increase the buckling and may cause concommitant damage to the surrounding tissue. The guide wire may further damage tissue when it is withdrawn. In some cases the user may be able to reposition the introducer but in others it will be necessary to repeat the operation with the possibility of further damage and trauma to the patient.

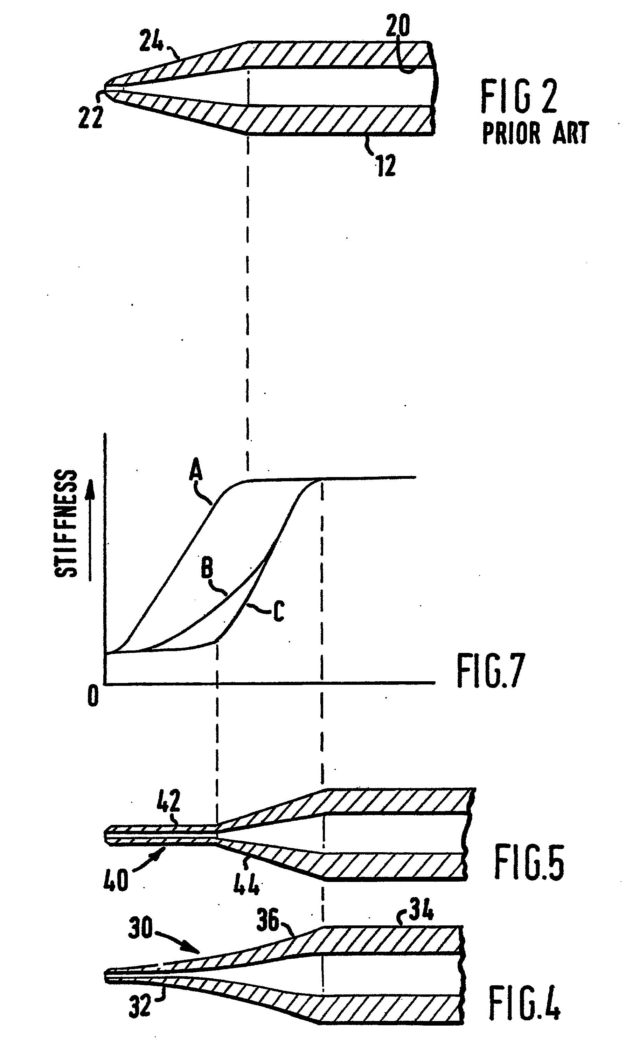

Figure 4 shows a dilator 30, according to the present invention. The tip of the dilator has a snout 32 integrally formed therewith but which is flexible by virtue of its thin wall. The snout 32 tapers gradually outward to the main wall 34 of the introducer. The taper of the snout is less than the included angle of the tip of the prior art dilator.

The alternative dilator of Figure 5 has a snout 42 of substantially constant inside and outside diameter which merges smoothly into the dilator 44.

Use of the present invention is illustrated in Figure 6. The flexible snout of the dilator ensures that the introducer follows the guide wire through the puncture wound. Because the outside diameter of the snout is only slightly greater than the guide wire, the initial insertion force is less and the chance of inadvertent tissue damage is much reduced. The snout provides support for the guide wire over a greater length and there is consequently a reduced chance of the guide wire kinking or buckling. The snout guides the dilator through the puncture wound ensuring minimal damage and patient pain. As the user uses greater force to insert the dilator, the flexural rigidity of the guide wire is effectively increased by the closely fitting snout to ensure that buckling is obviated.

The difference in stiffness of the tip of the dilators shown in Figures 2, 4 and 5 is illustrated for comparison purposes in the graph of Figure 7. The horizontal axis of the graph represents the increasing axial dimension of the respective dilators from the distal end; dotted lines to the figures indicate points which approximately correspond. The prior art dilator (Figure 2) is labelled A and shows a sharp increase in stiffness in a proximal direction. The dilators of Figures 4 and 5, labelled B and C respectively, show the comparatively low flexural rigidity of the snout.

The graph of Figure 7 is for illustration purposes only and is not intended to be scaled to provide proportionate relationships between the curves depicted or with the axes.

The length of the snout is dependent on the surgical procedure for which the introducer is intended although, as will be apparent from Figure 6, the snout should be sufficiently long to ensure that the introducer is guided into the puncture wound before the user applies increased force to dilate the opening. The snout should also be sufficiently long to ensure that the introducer follows the guide wire smoothly.

The flexural rigidity of the snout, as compared with the flexural rigidity of the main body of the introducer will be dependent on the materials used and the relative thickness and outside diameter. The material and relative dimensions will be chosen to suit the intended surgical procedure.

Other variations and modifications of the invention are possible and it is intended that the invention be limited only by the scope of the claims appended hereto.

## Claims

1. A catheter introducer for use with a guide wire and comprising a tubular body having an inwardly tapered tip (36, 44), characterised by said tip having a flexible snout (32, 42) adapted to follow the path of a guide wire (18).

2. An introducer according to Claim 1, wherein said snout is integrally formed with said tip.

3. An introducer according to Claim 1 or Claim 2, wherein said snout (42) has a substantially constant outside diameter.

4. An introducer according to any preceding claim, wherein said snout (42) has a substantially constant wall thickness.

5. A catheter introducer assembly comprising an introducer according to any preceding claim, a guide wire for the introducer and a sheath for the introducer.

## Patentansprüche

1. Kathetereinführvorrichtung zur Verwendung mit einem Führungsdraht, und einen rohrförmigen Körper mit einer einwärts verjüngten Spitze (36, 44) aufweisend, dadurch gekennzeichnet, daß die Spitze ein flexibles Mundstück (32, 42) aufweist, daß dem Pfad eines Führungsdrahts (18) folgen kann.

2. Einführvorrichtung nach Anspruch 1, bei der das Mundstück integral mit der Spitze geformt ist.

3. Einführvorrichtung nach Anspruch 1 oder 2, bei der das Mundstück (42) einen im wesentlichen konstanten Außendurchmesser hat.

4. Einführvorrichtung nach einem der vorhergehenden Ansprüche bei der das Mundstück (42) eine im wesentlichen konstante Wandstärke hat.

5. Kathetereinführanordnung mit einer Einführvorrichtung nach einem der vorhergehenden Ansprüche, einem Führungsdraht für die Einführvorrichtung und einer Umhüllung für die Einführvorrichtung.

## Revendications

1. Dispositif d'introduction pour cathéter destiné à être utilisé avec un fil de guidage et comprenant un corps tubulaire comportant un embout conique vers l'intérieur (36, 44), caractérisé en ce que l'embout présente un bec souple (32, 42) propre à suivre la voie empruntée par un fil de guidage (18).

2. Dispositif d'introduction suivant la revendication 1, dans lequel le bec est d'une pièce avec l'embout.

3. Dispositif d'introduction suivant la revendication 1 ou 2, dans lequel le bec (42) a un diamètre extérieur en substance constant.

4. Dispositif d'introduction suivant l'une quelconque des revendications précédentes, dans lequel le bec (42) a une épaisseur de paroi en substance constante.

5. Ensemble de dispositif d'introduction pour cathéter comprenant un dispositif d'introduction suivant l'une quelconque des revendications précédentes, un fil de guidage pour le dispositif d'introduction et une gaine pour ce dispositif.

FIG.1

FIG.3

FIG.6

**FIG 2**
**PRIOR ART**

**FIG.7**

STIFFNESS

A

B

C

0

**FIG.5**

**FIG.4**